# FASCICULE DE BREVET EUROPEEN

(11) **EP 0 580 490 B1**
(45) Date de publication et mention de la délivrance du brevet: **29.12.1997**
(21) Numéro de dépôt: 93401858.1
(22) Date de dépôt: 19.07.1993
(51) Int. Cl.: C12P 19/24, C07C 31/26

(54) **Procédé de fabrication de mannitol**
Verfahren zur Herstellung von Mannitol
Method of preparing mannitol

(30) Priorité: 22.07.1992 FR 9209053
(43) Date de publication de la demande: 26.01.1994
(73) Titulaire: Roquette Frères, F-62136 Lestrem (FR)
(72) Inventeur: Devos, Francis, F-59190 Morbecques (FR)
(74) Mandataire: Boulinguiez, Didier

(56) Documents cités:
- EP-A- 0 132 557
- US-A- 4 029 878

## Description

La présente invention concerne un procédé d'obtention de mannitol.

L'hydrogénation catalytique du mannose s'effectue avec un rendement stoechiométrique et fournit le mannitol.

L'hydrogénation catalytique du fructose permet aussi d'obtenir le mannitol, mais le rendement n'est pas stoechiométrique puisque l'hydrogénation du fructose n'est pas stéréospécifique et fournit du sorbitol en quantité égale.

Le mannitol est utilisé largement dans la fabrication du chewing-gum, des bonbons ou des excipients pharmaceutiques sans sucre. Cependant, l'obtention de mannose de pureté élevée est extrêmement difficile à réaliser et elle est onéreuse et la fabrication du mannitol n'est généralement réalisée industriellement qu'à partir de sirops de fructose, ce qui génère une quantité considérable de sorbitol.

Un procédé ancien consistait à hydrogéner un sirop de saccharose inverti, constitué à parts égales de glucose et de fructose et ne permettait d'obtenir qu'un sirop d'une teneur de 25 % de mannitol environ.

Un procédé plus récent décrit dans le brevet américain US-4 029 878, déposé en 1975, a consisté à procéder à l'épimérisation par voie chimique du glucose en mannose, l'hydrogénation de ce sirop épimérisé fournissant des sirops d'une teneur de 30 % de mannitol environ.

Une amélioration de ce procédé décrite dans le brevet américain US-4 173 514 déposé en 1977, a consisté à isomériser ces sirops épimérisés pour transformer une partie du glucose non épimérisé en fructose. Les équilibres thermodynamiques de ces réactions et les conditions industrielles de leur mise en oeuvre conduisent à l'obtention de mélanges ternaires glucose/mannose/fructose dans des proportions respectives d'environ 44/28/28 permettant d'obtenir par hydrogénation des sirops d'une teneur en mannitol d'environ 42 %.

L'hydrogénation des sirops à haute teneur en fructose conduit quant à elle à des sirops d'une teneur d'environ 48 % en mannitol, et l'hydrogénation de fructose pur conduirait à des sirops d'une teneur en mannitol d'environ 50 %.

Cependant, ces matières premières sont relativement onéreuses et ne permettent pas de fabriquer le mannitol dans les meilleures conditions économiques.

Tous ces procédés ont en outre pour inconvénient de générer une quantité considérable d'un sous-produit contenant essentiellement du sorbitol, mais contenant aussi du mannitol qu'on ne peut arriver à cristalliser et dont la perte amenuise encore les rendements. C'est ainsi que dans l'ordre où ils ont été cités précédemment, les procédés décrits ci-dessus ne permettent d'obtenir respectivement que environ 12 %, 17,5 %, 32 %, 39 % et 40 % de mannitol cristallisé pur par rapport aux sucres mis en oeuvre à l'étape d'hydrogénation catalytique.

Cela signifie aussi que même le procédé offrant le rendement le plus élevé, c'est-à-dire celui consistant à hydrogéner du fructose, génère cependant 1,58 kg d'un sous-produit difficilement valorisable contenant 85 % de sorbitol et 15 % de mannitol pour chaque kilogramme de mannitol cristallisé chimiquement pur produit par ce procédé.

Ce dernier procédé souffre également de l'inconvénient de nécessiter la mise en oeuvre d'une matière relativement chère : les sirops à très haute teneur en fructose. Quoique le plus performant en termes de rendement, il demandait donc à être amélioré.

Il y a de nombreuses années, PALLERONI et DOUDOROFF ont isolé d'un Pseudomonas saccharophila une enzyme qu'ils nommèrent mannose isomérase, capable d'interconvertir le fructose et le mannose dans les proportions respectives de 71/29 (J. Biol. Chem., 218, pages 535 à 548; 1956).

Une enzyme semblable a aussi été isolée par TAKASAKI d'un Streptomyces aerocolorigenes (Agr. Biol. Chem., Vol. 31, n° 4, pages 435 à 440; 1967) et par ANDERSON et ALLISON (J. Biol. Chem. 240, pages 2367 à 2372; 1965) d'un Aerobacter aerogenes sous le nom de D-Lyxose isomérase. Beaucoup plus tard, ALLENZA (US Patent n° 5 049 494 déposé en 1989) utilisa une mannose isomérase de Pseudomonas cepacia, extraite après rupture des parois cellulaires puis immobilisée sur un support d'alumine imprégné de polyéthylène imine et réticulée au glutaraldéhyde, ceci afin d'isomériser de façon continue une matière première contenant du mannose tele que les lessives résiduaires de papeterie, dans le but de fabriquer économiquement du fructose. Il émit alors l'hypothèse que le fructose formé pourrait être séparé du mannose par une technique chromatographique ou par séparation à l'aide de membranes. Toutefois, pour ce qui concerne l'obtention de mannose pur, matière première préférable pour la réalisation de son invention, il préconisait d'isoler celui-ci des autres sucres par précipitation de son dérivé bisulfitique ou du méthylmannoside, aveu manifeste du peu d'efficacité de ces techniques chromatographiques ou membranaires.

En 1982, HORWATH (brevet américain US-4 492 755) avait isomérisé le L-fructose en L-mannose à l'aide d'une L-mannose isomérase produite par un mutant de Klebsiella aerogenes et séparait le mannose formé et le fructose résiduel par cristallisation sélective des dérivés d'addition de ces sucres avec la phénylhydrazine et régénération du mannose à partir de sa phénylhydrazone.

En 1981 KRUSE et al (demande de brevet européen n° 74 713) préconisaient quant à eux d'enrichir le mannose contenu dans un sirop de glucose par précipitation sous forme d'un complexe mannose/chlorure de sodium.

Tous ces procédés de purification du mannose font intervenir des dérivés chimiques et sont difficilement industrialisables puisqu'il faut ensuite régénérer le mannose.

La séparation chromatographique de mélanges de glucose et de fructose a été décrite depuis longtemps et de nombreuses fois, aussi bien sur résines cationiques que sur zéolithes. Elle permet de préparer les sirops à très haute teneur en fructose. La fraction de glucose enrichi est toujours éluée un peu avant la fraction de fructose enrichi. On consultera ainsi avec intérêt le brevet américain n° 3 044 904 déposé en 1960 par CENTRAL AGUIRE SUGAR COMPANY, pour ce qui concerne la chromatographie des mélanges de glucose et de fructose sur des résines cationiques, et le brevet américain US 4 226 977 déposé en 1976 par UNION OIL PRODUCTS, pour ce qui concerne la chromatographie de ces mélanges sur des zéolithes.

La séparation chromatographique de mélanges de glucose et de mannose a également été décrite depuis longtemps et de nombreuses fois, sur résines cationiques comme sur zéolithes, dans le but essentiellement de produire des sirops à haute teneur en mannose, aptes à fournir par hydrogénation le mannitol avec un rendement élevé. Toutefois, la séparation chromatographique de ces deux sucres s'avère très peu efficace. Dans ce cas aussi, la fraction de glucose enrichi est toujours éluée un peu avant la fraction de mannose enrichi. On consultera avec intérêt le brevet anglais n° 1 540 556 déposé en 1977 par IMPERIAL CHEMICAL INDUSTRIES, pour ce qui concerne la chromatographie des mélanges de glucose et de mannose sur des résines cationiques, et la demande de brevet européen n° 115 631 déposée en 1983 par UNION CARBIDE, pour ce qui concerne la chromatographie de ces mélanges sur des zéolithes.

La demande de brevet européen n° 302 970 déposée en 1987 par UNION OIL PRODUCTS, relative à la séparation de différents aldohexoses et cétohexoses montre d'ailleurs clairement que si le glucose se sépare un peu du mannose et davantage du fructose sur des zéolithes, mannose et fructose ne se séparent pratiquement pas. La sélectivité fructose/mannose de l'adsorbant n'est en effet que de 1,68 alors qu'elle est de 4,84 pour fructose/glucose et de 2,88 pour mannose/glucose (4,84 : 1,68).

Si une sélectivité des adsorbants de l'ordre de 5 a permis une véritable explosion de la fabrication des sirops de fructose par chromatographie des mélanges glucose/fructose, une sélectivité de l'ordre de 3 s'est cependant avérée insuffisante pour permettre le développement de l'obtention de sirops à haute teneur en mannose à partir de mélanges glucose/mannose, ces sirops à haute teneur en mannose devant cependant constituer la matière première idéale permettant d'obtenir le mannitol avec un rendement quasi-stoechiométrique.

Or, la Société Demanderesse a eu le mérite de trouver qu'une sélectivité aussi basse que 1,68 pouvait cependant être exploitée dans un procédé de fabrication de sirops à haute teneur en mannose, destinés tout particulièrement à la fabrication de mannitol par hydrogénation catalytique, dès lors que les sirops soumis à la séparation chromatographique présentent à la fois une teneur en fructose d'au moins 65 %, une teneur en mannose d'au moins 15 % et une teneur en glucose inférieure à 15 %. Ces teneurs, ainsi que les teneurs apparaissant dans la suite de la description de la présente demande de brevet sont, sauf indications contraires, exprimées en poids par rapport à la matière sèche.

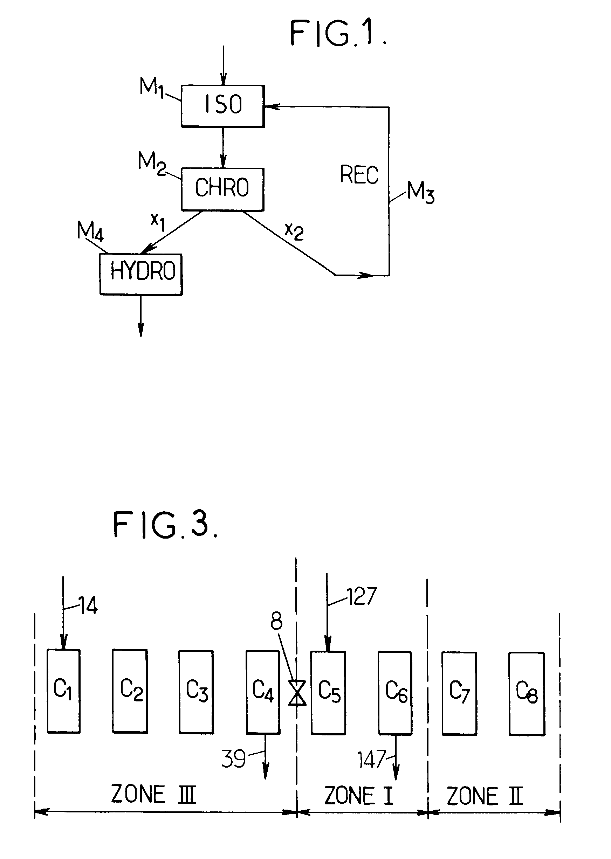

Le procédé de fabrication du mannitol conforme à l'invention comprend les étapes suivantes reprises schématiquement en figure 1 :
- dans une première étape M1, un sirop de fructose contenant moins de 15 % de glucose est soumis à une isomérisation enzymatique conduisant à l'obtention d'un mélange de fructose et de mannose contenant au moins 65 % de fructose et au moins 15 % de mannose.
- dans une deuxième étape M2, le susdit mélange est soumis à un traitement de séparation chromatographique conduisant à au moins deux fractions dont l'une est fortement enrichie en mannose (fraction X1) et dont l'autre est fortement enrichie en fructose (fraction X2).
- dans une troisième étape M3, la fraction X2 est, au moins partiellement, recyclée à l'étape d'isomérisation.
- dans une quatrième étape M4, la fraction X1 fortement enrichie en mannose est soumise à une hydrogénation catalytique.

Le sirop de fructose peut être obtenu de manière connue en soi par chromatographie de mélanges de glucose/fructose provenant de saccharose inverti ou d'hydrolysats d'amidon isomérisés. Il peut aussi être obtenu par hydrolyse de l'inuline. Dans les deux cas, il convient que ce sirop ne contienne pas plus de 15 % de glucose qui, se séparant mal du mannose, viendrait polluer de façon néfaste la fraction fortement enrichie en mannose issue de l'étape chromatographique.

Ce sirop contient plus de 80 %, de préférence plus de 85 % et plus préférentiellement encore plus de 90 % de fructose.

Selon une variante avantageuse du procédé selon l'invention, le sirop de fructose servant de matière première est un sirop obtenu par chromatographie d'hydrolysats d'amidons isomérisés et il contient plus de 95 % de fructose.

L'isomérisation enzymatique du fructose en mannose est effectuée de manière continue ou discontinue selon des procédés connus en eux-mêmes et décrits par exemple par PALLERONI et DOUDOROFF (J. Biol. Chem., 218, pages 535 à 548; 1956), pour ce qui concerne l'isomérisation discontinue et par ALLENZA (brevet américain n° 5 049 494), pour ce qui concerne l'isomérisation continue par enzyme immobilisée.

Un moyen commode est de laisser agir une boue bactérienne obtenue par centrifugation ou filtration d'un moût de culture de Pseudomonas saccharophila sur un sirop de fructose d'une matière sèche de 40 % environ à un pH de 7,5 environ et à une température de 30 à 50°C, et de préférence de 40°C environ. Il faut veiller dans ce cas à introduire les bactéries en quantité suffisante pour que la réaction d'isomérisation se déroule à une vitesse telle qu'on atteigne un pourcentage d'au moins 15 % de mannose en 24 à 30 heures au maximum, faute de quoi on se verrait contraint d'introduire un agent antiseptique dans le réacteur d'isomérisation. La découverte de mannose isomérase plus thermostable permettrait d'éviter cette contrainte.

Avantageusement, on procède à l'isomérisation du fructose en mannose de manière continue. Dans ce cas l'enzyme peut être extraite des bactéries par sonication de celles-ci et l'extrait brut peut être adsorbé ou fixé par liaison covalente sur un support inorganique activé comme cela est décrit dans le brevet américain US 5 049 494.

Un autre procédé d'immobilisation de l'enzyme, et qui est préférentiellement utilisé dans le procédé conforme à l'invention, consiste à immobiliser les cellules complètes au sein d'un réseau protéique constitué par exemple par de la gélatine réticulée à l'aide d'un agent pontant bifonctionnel comme le glutaraldéhyde. On trouve une description d'un tel mode d'immobilisation des cellules bactériennes dans le brevet américain US 4 163 691 dont la Société demanderesse est titulaire. Un tel procédé immobilise l'ensemble du potentiel enzymatique inclus à l'intérieur des cellules mais dans ce cas, l'absence de production de composés indésirables est due à la pureté du substrat qui ne contient essentiellement que du fructose.

Il faut toutefois veiller à ce que la masse microbienne immobilisée ne contienne pas de glucose isomérase en quantité sensible , faute de quoi une partie du fructose serait isomérisée en glucose, ce qui nuirait grandement à l'efficacité du procédé. Si tel est toutefois le cas, on ajoute un peu de calcium au sirop de fructose, ce qui inhibe la glucose isomérase sans affecter la mannose isomérase.

On évite également de procéder à l'isomérisation à un pH trop supérieur à 7,5 car dans ce cas une partie du fructose serait isomérisée en glucose par catalyse chimique alcaline.

De façon préférée, l'enzyme immobilisée est utilisée dans un réacteur en forme de colonne, calorifugée ou thermostatée et munie d'un fond poreux. On percole le sirop de fructose au travers des particules de cette enzyme à une température de 30 à 50°C à un débit tel que l'on obtienne en sortie une proportion de mannose comprise entre 15 et 29 %.

L'activité enzymatique emprisonnée dans le réacteur d'isomérisation est telle que sa concentration permette d'obtenir le pourcentage de mannose susmentionné avec un temps de séjour du sirop dans le réacteur de préférence compris entre 2 et 20 heures.

Le sirop de fructose enrichi en mannose après l'étape d'isomérisation est alors soumis au fractionnement chromatographique. Ce fractionnement chromatographique est effectué de manière connue en soi, de façon discontinue ou continue (lit mobile simulé), sur des adsorbants du type résines cationiques fortement acides, chargées préférentiellement à l'aide d'ions alcalins ou alcalinoterreux ou sur des zéolithes cationiques chargées avec les mêmes ions.

Des exemples de tels procédés sont décrits notamment dans les brevets US 3 044 904, 3 416 961, 3 692 582, FR 2 391 754, 2 099 336, US 2 985 589, 4 024 331, 4 226 977, 4 293 346, 4 157 267, 4 182 633, 4 332 623, 4 405 455, 4 412 866 et 4 422 881.

Selon un mode de réalisation préféré, le fractionnement chromatographique est réalisé en employant le procédé et appareillage décrits dans le brevet américain US 4 422 881 dont la Société demanderesse est titulaire.

Quel que soit le procédé chromatographique retenu, on a recours de préférence en ce qui concerne l'adsorbant, à une résine cationique forte employée sous forme calcium et réticulée avec environ 4 à 10 % de divinylbenzène. Les résines sont avantageusement de granulométrie homogène et comprise entre 100 et 800 microns.

Le choix des paramètres du fractionnement chromatographique, parmi lesquels on note plus particulièrement le débit d'élution, le débit d'alimentation en sirop de fructose isomérisé, le débit d'extraction de la fraction enrichie en mannose, le débit de la fraction enrichie en fructose et la composition des zones de désorption, d'adsorption et d'enrichissement est expliqué et illustré dans les exemples.

Le choix de ces paramètres est effectué de telle manière que la fraction X1 ait une richesse en mannose, les pourcentages étant exprimés en poids par rapport à la matière sèche, comprise entre 30 et 80 %, de préférence entre 35 % et 70 % et plus préférentiellement encore entre 40 % et 60 %, ainsi qu'une richesse en glucose inférieure à 15 % et de préférence inférieure à 10 %.

Pour parvenir à ce résultat, ces paramètres sont choisis comme suit quand le fractionnement chromatographique est réalisé en utilisant les procédé et appareillage décrits dans le brevet US 4 422 881 et quand l'adsorbant utilisé est une résine cationique de faible granulométrie réticulée à 6 % de divynylbenzène et qu'elle est utilisée sous forme calcium :
- débit d'élution de 70 à 700 l/h/m3 d'adsorbant,
- débit d'alimentation en sirop isomérisé de 10 à 100 l/h/m3 d'adsorbant,
- débit d'extraction de la fraction enrichie en mannose : de 80 à 800 l/h/m3 d'adsorbant,
- débit d'extraction de la fraction enrichie en fructose : 20 à 200 l/h/m3 d'adsorbant.

L'étape de fractionnement chromatographique amène la production conjointe de la fraction X2, fortement enrichie en fructose, et éventuellement d'une fraction X3 contenant du glucose existant comme impureté dans les sirops de fructose et dont il convient d'effectuer la purge périodique afin que le glucose ne s'accumule pas et que sa teneur ne dépasse pas, après recyclage de la fraction X2 dans le sirop de fructose utilisé comme matière première, une valeur de 15 % dans le sirop soumis au fractionnement chromatographique.

La fraction X2, fortement enrichie en fructose, présente de préférence la composition suivante, les pourcentages étant exprimés en poids par rapport à la matière sèche:
- de 76 à 97 % de fructose,
- de 2 à 24 % de mannose,
- de 0 à 15 % de glucose.

Conformément à l'invention, cette fraction X2 fortement enrichie en fructose est recyclée vers l'étape d'isomérisation.

Grâce au procédé conforme à l'invention qui tire profit des bénéfices combinés de l'isomérisation du fructose, de la séparation chromatographique du sirop isomérisé et du recyclage du fructose séparé non isomérisé, il devient possible de convertir avec un très fort rendement le fructose en mannose.

La richesse en mannose de la fraction X1, insuffisante en elle-même pour donner par hydrogénation du mannitol avec un bon rendement, s'avère cependant suffisante dans la mesure où l'impureté accompagnatrice, le fructose, génère elle aussi du mannitol par hydrogénation, compensant ainsi la faible sélectivité fructose/mannose de l'adsorbant.

Qui plus est, les richesses en mannitol atteintes après l'étape d'hydrogénation permettent de cristalliser celui-ci à l'état pur en une seule opération de cristallisation. Il est même possible en séchant complètement cette fraction X1 d'obtenir avec un rendement total un mannitol technique de pureté très élevée. Les procédés connus jusqu'alors, y compris le procédé d'hydrogénation directe des sirops de fructose isomérisés, ne permettaient pas d'obenir une richesse en mannitol suffisante et obligeaient à procéder à plusieurs recristallisations successives du mannitol.

C'est ainsi qu'un sirop à 25 % de mannitol obtenu à partir de saccharose par exemple obligeait à pratiquer trois cristallisations successives et qu'un sirop d'une richesse inférieure à 65 % de mannitol obligeait à pratiquer deux cristallisations successives.

L'un des avantages essentiels du procédé selon l'invention réside donc dans le fait qu'il permet d'obtenir le mannitol cristallisé chimiquement pur dès lors que la richesse en mannose de la fraction X1 est supérieure à 30 %, ce qui se traduit par une richesse en mannitol après hydrogénation supérieure à 65 %.

Grâce au procédé selon l'invention, et en pratiquant plusieurs cristallisations successives sur les eaux mères, ce qui est rendu possible par la haute richesse des sirops obtenus après hydrogénation, le rendement en mannitol cristallisé par rapport au sucre (fructose) mis en oeuvre peut atteindre 70 % et davantage, la production de 1 kg de mannitol ne générant plus que la production de 0,430 kg d'un sous-produit contenant 85 % de sorbitol, ce qui équivaut à une diminution d'un facteur de 3.3 de la production de sous-produit par rapport au meilleur procédé de l'art antérieur, utilisant le fructose comme matière première mais avec un rendement de 40 %.

### EXEMPLES

### ISOMERISATION

Une souche de pseudomonas saccharophila ATCC 15946 adaptée à la culture sur milieu au fructose a été cultivée dans les conditions décrites par PALLERONI et DOUDOROFF.

Les cellules ont été centrifugées et la boue bactérienne obtenue a été additionnée d'une solution de gélatine à 50 % de matières sèches dans des proportions de 100 kg de solution de gélatine pour 200 kg de boue.

Le produit résultant a été injecté dans une filière perforée de 400 trous de 500 microns de diamètre immergée dans l'eau froide, conformément au procédé décrit dans le brevet français 2 353 562.

La réticulation de la gélatine a été effectuée par contact de 15 heures des boues bactériennes ainsi filées dans une solution de glutaraldéhyde à 1 % maintenue à une température de 2°C. Les fils formés ont été découpés en morceaux d'une longueur moyenne de 2 mm et ont été immédiatement chargés dans des colonnes thermostatées à 35°C, d'une hauteur de 200 cm et d'un diamètre de 25 cm.

On a percolé sur ces colonnes un sirop de fructose obtenu par refonte de fructose cristallisé, présentant une matière sèche égale à 40 % et un pH de 7,0. Les colonnes ont été alimentées à un débit de 30 litres/heure de sirop de fructose et on a recueilli au bas de ces colonnes un sirop de fructose isomérisé contenant 25 % de mannose et 75 % de fructose.

Le sirop obtenu a été déminéralisé puis concentré à une matière sèche de 53 %.

### SEPARATION CHROMATOGRAPHIOUE

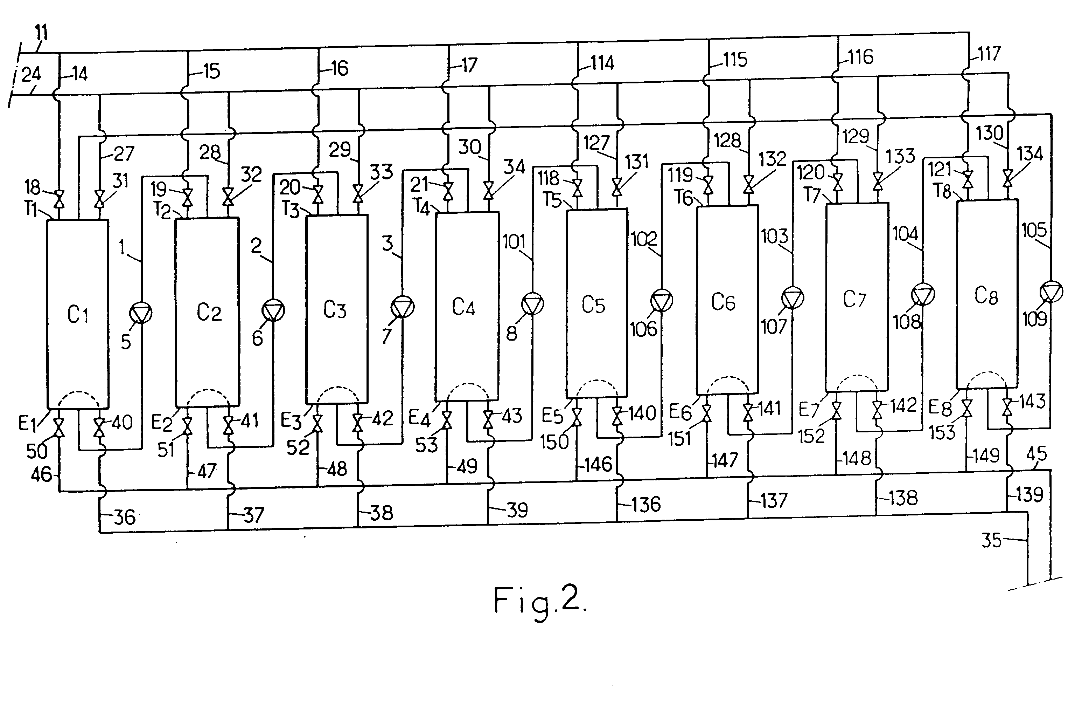

Le fractionnement du sirop isomérisé riche en mannose a été effectué dans l'installation de séparation chromatographique continue dont les détails de construction et de fonctionnement sont décrits dans le brevet américain US 4 422 881, ces détails n'étant repris ici que pour ce qui est nécessaire à la compréhension du procédé. Cette installation comprend, comme montré à la figure 2 du brevet américain (reprise ici en tant que figure 2 et pour l'explication détaillée de laquelle on se référera audit brevet américain), huit colonnes ou étages C 1 à C 8 de 200 litres chacun, remplis de résine cationique forte permutée sous forme calcium et d'une granulométrie comprise entre 200 et 400 microns du type DUOLITE C 204-2078.

De par le calage des électrovannes, on établit dans cette installation une zone I de désorption de deux étages, une zone II d'adsorption de deux étages et une zone III d'enrichissement et de séparation du mannose relativement moins adsorbé et du fructose relativement plus adsorbé de quatre étages comme montré sur la figure 3, qui est une représentation schématique de l'installation selon la figure 2 et sur laquelle on n'a fait figurer que
- les colonnes C 1 à C 8,
- le dispositif de fermeture, en l'occurrence l'électrovanne 8,
- les canalisations d'alimentation en eau et en sirop isomérisé à fractionner riche en mannose montrées respectivement en 127 et 14, et
- la canalisation 147 d'extraction de sirop enrichi en fructose (fraction X2) d'une part, et la canalisation 39 d'extraction du mannose (fraction X1).

Le dispositif de fermeture 8 maintient dans la configuration adoptéee une étanchéité totale entre, d'une part, la zone III, qui est une zone d'enrichissement à l'extrémité de laquelle est récupérée la fraction enrichie en mannose et d'autre part la zone I de désorption du fructose, zone en tête de laquelle est introduite l'eau de désorption.

Ce dispositif de fermeture assure le sens du passage de la phase liquide sur l'adsorbant sélectif.

Une minuterie ajustée à 1500 secondes assure pour les débits indiqués dans le tableau I une alimentation en eau sur la première colonne de la zone I de désorption de la totalité du fructose, et une alimentation en un volume de sirop isomérisé riche en mannose sur le premier étage de la zone III compatible avec le volume d'adsorbant et sa capacité d'adsorption, de façon à obtenir un rendement d'extraction du mannose au moins égal à 7,5 % du mannose présent dans le sirop isomérisé et cela à une richesse au moins égale à 40 % en mannose. Au terme des 1500 secondes, toutes les entrées et sorties, de même que le dispositif de fermeture 8 sont décalés d'un étage vers la droite.

Les susdits taux d'extraction et pureté sont maintenus constants en ajustant le débit de la pompe d'extraction, non montrée, du fructose adsorbé. La sortie de la fraction X1, éventuellement précédée d'une fraction X3 contenant un peu de glucose, s'effectue à pression atmosphérique et son débit constant résulte de la différence entre les débits d'alimentation et le débit d'extraction.

Le sirop isomérisé riche en mannose qui est introduit dans l'installation en tête de la zone d'enrichissement et de séparation III, présente une matière sèche de 53 %. La température à l'intérieur des colonnes de séparation est maintenue à environ 70°C.

La fraction X1 enrichie en mannose, ainsi qu'éventuellement la fraction X3, sont précédées d'une fraction d'eau pure ayant servi au désucrage de la colonne lors du cycle précédent. Cette fraction d'eau pure est avantageusement éliminée durant les dix-huit premières minutes de chaque cycle, ce qui permet d'accroître fortement la concentration des fractions de mannose enrichi.

Le tableau I ci-après montre pour différentes conditions de conduite de l'installation les richesses et concentrations obtenues pour les deux fractions X1 et X2.

Les conditions préférées pour la conduite du fractionnement chromatographique sont celles pour lesquelles la teneur en mannose de la fraction X1 est comprise entre 40 et 60 %. Une telle richesse en mannose est suffisante pour fournir après hydrogénation des sirops d'une teneur supérieure à 70 % de mannitol, richesse qu'il n'est pas indispensable de dépasser pour obtenir le mannitol cristallisé pur sans avoir à effectuer une recristallisation.

En outre, dans ces conditions, le rendement d'extraction du mannose, que l'on peut exprimer comme étant le rapport du poids de mannose pur extrait dans la fraction X1 sur le poids de mannose pur contenu dans le sirop d'alimentation, est beaucoup plus élevé.

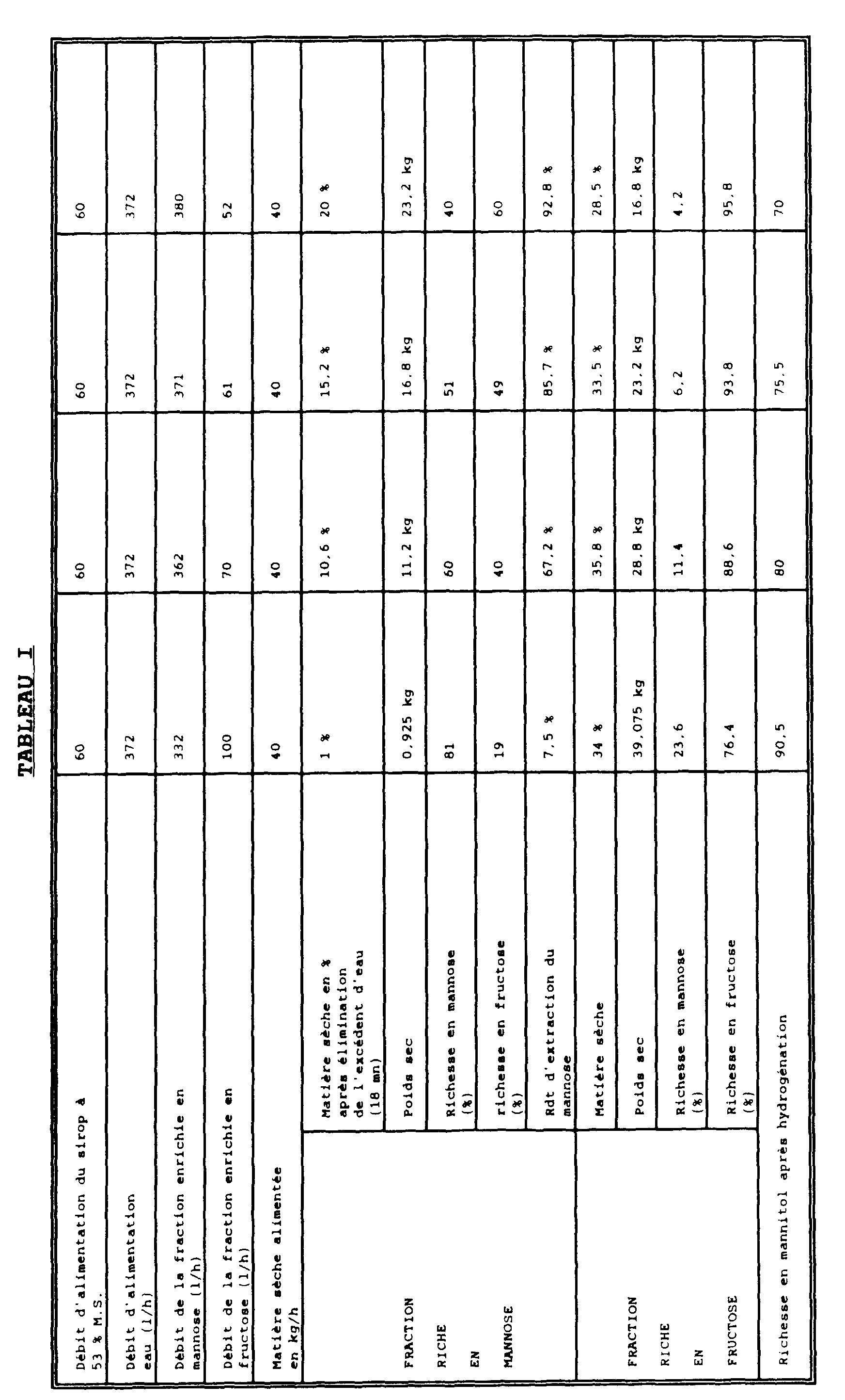

La fraction de fructose enrichie peut être isomérisée, dans les conditions déjà décrites précédemment, de façon séparée du sirop de fructose servant de matière première, mais elle peut aussi être mélangée à cette matière première et ce mélange peut être isomérisé comme un tout.

La fraction X1 riche en mannose est hydrogénée de façon connue en soi. Elle est d'abord concentrée à une matière sèche de 40 % puis hydrogénée à une température de 140°C sous une pression d'hydrogène de 50 bars en présence de catalyseur au NICKEL de RANEY.

On a obtenu dans tous les cas des sirops dont la richesse en mannitol est supérieure à 70 %, comme cela figure également dans le tableau I.

## Revendications

1. Procédé de fabrication de mannitol, caractérisé par le fait :
- que dans une première étape on soumet un sirop de fructose contenant moins de 15 % de glucose à une isomérisation enzymatique à l'aide d'au moins une mannose isomérase capable d'interconvertir le fructose et le mannose, ladite isomérisation enzymatique conduisant à l'obtention d'un mélange de fructose et de mannose contenant au moins 65 % de fructose et au moins 15 % de mannose,
- que dans une seconde étape, on soumet ledit mélange à un traitement de séparation chromatographique de façon à obtenir au moins deux fractions dont l'une (fraction X1) est fortement enrichie en mannose et dont l'autre (fraction X2) est fortement enrichie en fructose,
- que dans une troisième étape, on recycle au moins partiellement la fraction X2 en tête de l'étape d'isomérisation enzymatique,
- que dans une quatrième étape, on hydrogène la fraction X1 fortement enrichie en mannose de façon à obtenir un sirop riche en mannitol.

2. Procédé selon la revendication 1, caractérisé par le fait que le sirop de fructose contient plus de 80 %, de préférence plus de 85 %, et plus préférentiellement encore plus de 90 % de fructose.

3. Procédé selon l'une ou l'autre des revendications 1 et 2, caractérisé par le fait que le mélange soumis au traitement de séparation chromatographique présente une teneur en fructose d'au moins 65 %, une teneur en glucose inférieure à 15 %, et une teneur en mannose supérieure à 15 %, de préférence comprise entre 15 et 29 %.

4. Procédé selon l'une quelconque des revendications 1 à 3, caractérisé par le fait que l'isomérisation enzymatique du sirop de fructose est effectuée de manière discontinue ou continue à un pH d'environ 7.5 et à une température de 30 à 50° C, et de préférence en présence d'ions calcium.

5. Procédé selon l'une quelconque des revendications 1 à 4, caractérisé par le fait que l'on effectue le traitement chromatographique dans des conditions telles que la fraction X1 présente une richesse en mannose comprise entre 30 et 80 %, de préférence entre 35 et 70 % et plus préférentiellement encore entre 40 et 60 %, ainsi qu'une richesse en glucose inférieure à 15 % et de préférence inférieure à 10%.

6. Procédé selon l'une quelconque des revendications 1 à 5, caractérisé par le fait que l'on effectue le traitement chromatographique dans des conditions telles que la fraction X2 présente une teneur en fructose comprise entre 76 et 97 %, une teneur en mannose comprise entre 2 et 24 % et une teneur en glucose comprise entre 0 et 15 %;

7. Procédé selon l'une quelconque des revendications 1 à 6, caractérisé par le fait que le sirop riche en mannitol obtenu présente une richesse en mannitol supérieure à 65 %.

8. Procédé selon l'une quelconque des revendications 1 à 7, caractérisé par le fait que l'on soumet ultérieurement le sirop riche en mannitol à une seule étape de cristallisation de façon à obtenir du mannitol cristallisé.

## Patentansprüche

1. Verfahren zur Herstellung von Mannitol, dadurch gekennzeichnet, daß
- man in einer ersten Stufe einen Fructosesirup, der weniger als 15 % Glucose enthält, einer enzymatischen Isomerisierung mit Hilfe von mindestens einer Mannose-Isomerase unterzieht, die fähig ist, Fructose und Mannose zu interkonvertieren, wobei die genannte enzymatische Isomerisierung zum Erhalten einer Mischung von Fructose und Mannose führt, die mindestens 65 % Fructose und mindestens 15 % Mannose enthält,
- man in einer zweiten Stufe die genannte Mischung einer Behandlung zur chromatographischen Trennung unterzieht, so daß man mindestens zwei Fraktionen erhält, von denen eine (Fraktion X1) stark mit Mannose und die andere (Fraktion X2) stark mit Fructose angereichert ist,
- man in einer dritten Stufe mindestens teilweise die Fraktion X2 an den Anfang der Stufe zur enzymatischen Isomerisierung zurückführt,
- man in einer vierten Stufe die stark mit Mannose angereicherte Fraktion X1 hydriert, so daß man einen Sirup erhält, der reich an Mannitol ist.

2. Verfahren nach Anspruch 1, dadurch gekennzeichnet, daß der Fructosesirup mehr als 80 %, vorzugsweise mehr als 85 % und ganz besonders bevorzugt mehr als 90 % Fructose enthält.

3. Verfahren nach dem einen oder anderen der vorstehenden Ansprüche, dadurch gekennzeichnet, daß die Mischung, die einer Behandlung zur chromatographischen Trennung unterzogen wird, einen Gehalt an Fructose von mindestens 65 %, einen Gehalt an Glucose von unter 15 % und einen Gehalt an Mannose von höher als 15 %, vorzugsweise zwischen 15 % und 29 % aufweist.

4. Verfahren nach irgendeinem der Ansprüche 1 bis 3, dadurch gekennzeichnet, daß die enzymatische Isomerisierung des Fructosesirups in diskontinuierlicher oder kontinuierlicher Weise bei einem pH-Wert von etwa 7,5 und einer Temperatur von 30 °C bis 50 °C und vorzugsweise in Anwesenheit von Calciumionen durchgeführt wird.

5. Verfahren nach irgendeinem der Ansprüche 1 bis 4, dadurch gekennzeichnet, daß man die chromatographische Behandlung unter solchen Bedingungen durchführt, daß die Fraktion X1 eine Anreicherung an Mannose zwischen 30 % und 80 %, vorzugsweise zwischen 35 % und 70 % und ganz besonders bevorzugt zwischen 40 % und 60 %, sowie eine Anreicherung an Glucose von unter 15 % und vorzugsweise von unter 10 % aufweist.

6. Verfahren nach irgendeinem der Ansprüche 1 bis 5, dadurch gekennzeichnet, daß man die chromatographische Behandlung unter solchen Bedingungen durchführt, daß die Fraktion X2 einen Gehalt an Fructose zwischen 76 % und 97 %, einen Gehalt an Mannose zwischen 2 % und 24 % und einen Gehalt an Glucose zwischen 0 % und 15 % aufweist.

7. Verfahren nach irgendeinem der Ansprüche 1 bis 6, dadurch gekennzeichnet, daß der erhaltene, mit Mannitol angereicherte Sirup eine Anreicherung an Mannitol von höher als 65 % aufweist.

8. Verfahren nach irgendeinem der Ansprüche 1 bis 7, dadurch gekennzeichnet, daß man den mit Mannitol angereicherten Sirup später einer einzigen Stufe zur Kristallisation unterzieht, um kristallines Mannitol zu erhalten.

## Claims

1. Process for the manufacture of mannitol, characterised by the fact that:
- in a first step, a fructose syrup containing less than 15% glucose is subjected to an enzymatic isomerisation using at least one enzyme capable of interconverting fructose and mannose, the said enzymatic isomerisation resulting in the production of a mixture of fructose and mannose containing at least 15% mannose,
- in a second step, the said mixture is subjected to a chromatographic separation treatment so as to obtain at least two fractions one of which (fraction X1) is highly enriched with mannose and the other of which (fraction X2) is highly enriched with fructose,
- in a third step, fraction X2 is at least partially recycled to the top of the enzymatic isomerisation step,
- in a fourth step, fraction X1, highly enriched with mannose, is hydrogenated so as to obtain a mannitol-rich syrup.

2. Process according to Claim 1, characterised by the fact that the fructose syrup contains more than 80%, preferably more than 85% and still more preferably more than 90% fructose.

3. Process according to either of Claims 1 and 2, characterised by the fact that the mixture subjected to the chromatographic separation treatment has a fructose content of at least 65%, a glucose content of less than 15%, and a mannose content of greater than 15%, preferably of between 15 and 29%.

4. Process according to any one of Claims 1 to 3, characterised by the fact that the enzymatic isomerisation of the fructose syrup is carried out batchwise or continuously at a pH of about 7.5 and at a temperature of 30 to 50°C, and preferably in the presence of calcium ions.

5. Process according to any one of Claims 1 to 4, characterised by the fact that the chromatographic treatment is carried out under conditions such that fraction X1 has a mannose level of between 30 and 80%, preferably of between 35 and 70% and still more preferably of between 40 and 60%, as well as a glucose level of less than 15% and preferably less than 10%.

6. Process according to any one of Claims 1 to 5, characterised by the fact that the chromatographic treatment is carried out under conditions such that fraction X2 has a fructose content of between 76 and 97%, a mannose content of between 2 and 24% and a glucose content of between 0 and 15%.

7. Process according to any one of Claims 1 to 6, characterised by the fact that the mannitol-rich syrup obtained has a mannitol level greater than 65%.

8. Process according to any one of Claims 1 to 7, characterised by the fact that the mannitol-rich syrup is subsequently subjected to a single crystallisation step so as to obtain crystallised mannitol.
